# EUROPÄISCHE PATENTSCHRIFT

(11) **EP 2 996 647 B1**
(45) Veröffentlichungstag und Bekanntmachung des Hinweises auf die Patenterteilung: **27.09.2017**
(21) Anmeldenummer: 14727723.0
(22) Anmeldetag: 12.05.2014
(51) Int. Cl.: A61F 7/00, A47C 21/04, H05B 1/02, A61G 13/00

(54) **ANORDNUNG ZUM ERWÄRMEN EINER PATIENTENLAGERFLÄCHE**
ARRANGEMENT FOR WARMING A PATIENT SUPPORT SURFACE
SYSTÈME DE CHAUFFAGE D'UNE SURFACE DESTINÉE À SUPPORTER UN PATIENT

(30) Priorität: 14.05.2013 DE 102013104931
(43) Veröffentlichungstag der Anmeldung: 23.03.2016
(73) Patentinhaber: Maquet GmbH, 76437 Rastatt (DE)
(72) Erfinder: KATZENSTEIN, Bernhard, Iffezheim 76473 (DE)
(74) Vertreter: Zacco GmbH
(86) Internationale Anmeldenummer: PCT/EP2014/059620
(87) Internationale Veröffentlichungsnummer: WO 2014/184128

(56) Entgegenhaltungen:
- WO-A2-03/088881
- WO-A2-2009/006517
- JP-A- 2001 238 924
- US-A- 5 881 410
- US-A1- 2002 156 509

## Beschreibung

Die Erfindung betrifft eine Anordnung zum Erwärmen einer Patientenlagerfläche, insbesondere zum Erwärmen von Polsterelementen einer Patientenlagerfläche eines Operationstischs. Die Anordnung umfasst ein erstes Polsterelement und mindestens ein zweites Polsterelement, die jeweils ein im Polsterelement integriertes elektrisches Heizelement haben.

Während einer medizinischen Behandlung und bei Operationen kann die Körpertemperatur eines Patienten durch die gegenüber der Körpertemperatur geringere Umgebungstemperatur während der Behandlung sinken. Eine solche Reduktion der Körpertemperatur, insbesondere der Kerntemperatur, des Patienten kann zu Komplikationen während und nach der Behandlung bzw. der Operation führen. Um dies zu verhindern, sind verschiedene Heizvorrichtungen zur Erwärmung bzw. zur Warmhaltung von Patienten bekannt, die den Wärmeverlust des Patienten während der Behandlung bzw. der Operation gering halten. Üblicherweise sind die Heizvorrichtungen jedoch nicht dafür vorgesehen, die Kerntemperatur des Patienten zu erhöhen, jedoch können bekannte Heizsysteme das Auskühlen des Körpers auf ein Minimum reduzieren.

Ferner werden diese Heizvorrichtungen aus unterschiedlichen Gründen, insbesondere während der Vor- und Nachbereitung von Operationen, nicht permanent betrieben, sodass während dieser Zeit die Kerntemperatur des Patienten abkühlen kann. Übliche Heizvorrichtungen bestehen beispielsweise aus Heizmatten, die präoperativ, d. h. vor dem Patienten, auf der Lagerfläche positioniert werden müssen. Eine Anpassung von modularen auswechselbaren Komponenten der Patientenlagerfläche an die Körpergröße des Patienten und an die Erfordernisse der Operation ist durch die zusätzlichen Heizmatten aufwendiger als ohne diese Heizmatten. Flüssigkeitsdurchströmte Heizmatten haben zusätzlich den Nachteil, dass sie ein relativ hohes Gewicht haben, da sie zumindest zum Teil mit Flüssigkeit gefüllt sind. Ferner beeinflusst eine ungleichmäßige Verteilung von Flüssigkeit in den Heizmatten Röntgenaufnahmen und die Liegeigenschaften der Patientenlagerfläche negativ.

Ferner ist es bei zusätzlich auf die Polsterelemente aufgelegten Heizmatten erforderlich, diese zumindest nach einer Operation durch Wischdesinfektion zusätzlich zu den darunterliegenden Polsterelementen zu reinigen. Dies verlängert die Aufbereitungszeit der Patientenlagerfläche nach einer Operation. Ferner bedarf die Lagerung der separaten Heizmatten erheblichen Lagerplatz, da Heizmatten und deren Zuleitungen üblicherweise nicht gefaltet oder geknickt werden dürfen.

Alternativ zu den bekannten Heizmatten besteht eine weitere Möglichkeit darin, den Patienten während der Operation in einem warmen Luftstrom anzuordnen. Alternativ kann eine Bestrahlung des Patienten mit Hilfe von Wärmestrahlern erfolgen. Diese Lösungen sind jedoch bei einer Operation insofern nachteilig, als dass auch die die Operation durchführenden Personen dieser Wärmestrahlung ausgesetzt sind.

Die Wärmeabgabe von bekannten Heizmatten kann durch eine beispielsweise an einem Infusionsständer fixierten separat angeordneten Steuereinheit gesteuert werden. Dadurch kann die vom Anwender gewünschte Solltemperatur einfach mit Hilfe dieser Steuereinheit eingestellt werden. Es kann eine Regelung mit Hilfe von in der Heizmatte angeordneten Temperatursensoren und/oder mit Hilfe von Sensoren, die die Körpertemperatur des Patienten erfassen, geregelt werden. Bei elektrischen Widerstandsheizmatten kann hierzu der Strom, der durch das Heizelement dieser Heizmatten fließt, oder die Einschaltzeit pro Zeiteinheit als Stellgröße beeinflusst werden.

Die Steuereinheit kann eine Bedienschnittstelle, ein sogenanntes Userinterface, zur Bedienung des aus Steuereinheit und Heizmatte bestehenden Heizsystems umfassen.

Aus Sicherheitsgründen kann die Spannungsversorgung der Heizmatte mit einer Betriebsspannung kleiner der Netzspannung erfolgen, beispielsweise mit Schutzkleinspannung. Die Steuereinheit kann zusätzlich einen Akkumulator umfassen, der bei Trennung der Steuereinheit von einem Versorgungsnetz die Energieversorgung der Heizmatte übernimmt. Vorzugsweise ist dieser Akkumulator in die Steuereinheit integriert und wird über eine entsprechende Ladeschaltung geladen, solange die Steuereinheit mit einem Versorgungsnetz verbunden ist.

Aus dem Dokument WO 2008/110922 A2 ist ein Polster mit einem integrierten Heizelement sowie Temperatursensoren zum Erfassen der Temperatur des Polsters bekannt. Ferner ist eine in das Polster integrierte Schaltung zur Übertragung der mit Hilfe der Temperatursensoren erfassten Temperaturwerte zu einer externen Steuereinheit bekannt. Aus dem Dokument US 5,888,410 ist eine Luftmatratze für einen Operationstisch bekannt. Ausgehend von dem bekannten Stand der Technik, ist es Aufgabe der Erfindung, eine Anordnung zum Erwärmen für eine Patientenlagerfläche, insbesondere zum Erwärmen von Polsterelementen für eine Patientenlagefläche eines Operationstischs, anzugeben, bei denen eine einfache und sichere Handhabung möglich ist.

Diese Aufgabe wird durch eine Anordnung zum Erwärmen einer Patientenlagerfläche mit den Merkmalen des Anspruchs 1 sowie durch einen in den Ansprüchen angegebenen Operationstisch gelöst. Vorteilhafte Weiterbildungen sind in den abhängigen Ansprüchen angegeben.

Im Unterschied zu einteiligen Heizmatten oder einstückigen Polsterelementen mit integrierten Heizelementen bleibt durch das Vorsehen mehrerer Polsterelemente, die jeweils ein im Polsterelement integriertes elektrisches Heizelement haben, die Verstellfunktion von Komponenten einer aus mehreren Komponenten zusammengesetzten Patientenlagerfläche eines Operationstischs uneingeschränkt erhalten. Ferner kann die Wärmeabgabe in unterschiedlichen Bereichen der Patientenlagefläche einfach, erforderlichenfalls auch unterschiedlich, eingestellt werden, wobei die Polsterelementsteuereinheiten die Wärmeabgabe des Heizelements des jeweiligen Polsterelements regeln. Dadurch wird eine hohe Genauigkeit bei der Wärmeabgabe der gewünschten Wärmemenge an den Patienten erreicht. Dabei ist der Regelkreis zum Regeln der Wärmeabgabe des Heizelements bzw. zum Regeln der Temperatur des Heizelements und/oder des Polsterelements vollständig in das Polsterelement integriert. Als Stellgröße wird dabei der durch das Heizelement fließende Strom eingestellt oder es erfolgt eine Pulsweitenmodulation der Versorgungsspannung zum Versorgen des Heizelements mit elektrischer Energie. Als Ist-Größe dient der mit Hilfe des Temperatursensors ermittelte Temperaturistwert. Dieser Temperatursensor kann ein passiver Temperatursensor, vorzugsweise ein Zwei-Draht-TemperaturSensor, sein. Als Temperatursensoren eignen sich insbesondere PT100 Sensorelemente, PTC-Thermistoren, vorzugsweise PTC100-Thermistoren, und NTC-Thermistoren. Die zentrale Steuereinheit hat vorzugsweise ein Userinterface zur Bedienung der zentralen Steuereinheit, insbesondere zum Einstellen der gewünschten Temperatursollwerte und/oder Heizleistungen. Ferner können über eine Anzeigeeinheit des Userinterfaces auch Informationen, wie zum Beispiel Fehlermeldungen, Statusmeldungen, Ist- und/oder Soll-Temperaturen, ausgegeben werden.

Durch die Integration der Heizelemente in die Polsterelemente ist ferner der Reinigungs- und Desinfektionsaufwand gegenüber zusätzlich zu den Polsterelementen vorgesehenen Heizmatten reduziert. Der Aufwand zur Demontage und Aufbewahrung von Heizmatten und Polsterelementen ist durch die Erfindung gegenüber separaten Polsterelementen und Heizmatten reduziert.

Die Heizleistung der Heizelemente der Polsterelemente ist derart dimensioniert, dass die an der Oberfläche der Patientenlagerfläche bewirkte Heizleistung einen maximalen Wert im Bereich von 90 bis 200 W/m², vorzugsweise einen Wert kleiner 115 W/m² hat. Hierdurch wird eine ausreichend schnelle Aufheizung der Polsterelemente und eine ausreichende Wärmezufuhr zu einem die Polsterelemente kontaktierenden Patienten erreicht.

Es ist vorteilhaft, wenn die zentrale Steuereinheit beiden Polsterelementsteuereinheiten denselben Temperatursollwert oder jeder Polsterelementsteuereinheit einen individuellen Temperatursollwert vorgibt. Dadurch ist eine einfache Handhabung bzw. eine individuelle Wärmeabgabe der einzelnen Polsterelemente möglich, sodass sich die Heizleistung gezielt an die Bedürfnisse des Patienten und die Anforderungen, die aus der durchzuführenden Behandlung des Patienten resultieren, einstellen lassen.

Ferner ist es vorteilhaft, wenn jede Polsterelementsteuereinheit den mit Hilfe des Temperatursensors ermittelten Temperaturistwert zur zentralen Steuereinheit überträgt. Hierzu ist vorzugsweise eine bidirektionale Datenübertragung zwischen der zentralen Steuereinheit und den Polsterelementsteuereinheiten vorgesehen. Durch die Übertragung der ermittelten Temperaturistwerte zur zentralen Steuereinheit kann eine einfache zentrale Temperaturüberwachung erfolgen. Ferner kann eine Anzeige der übermittelten Temperaturistwerten an einer Anzeigeeinheit der zentralen Steuereinheit und/oder die Generierung und Ausgabe von Fehlersignalen durch die zentrale Steuereinheit erfolgen. Ferner ist es besonders vorteilhaft, wenn die zentrale Steuereinheit zumindest einen Teil der von den Polsterelementsteuereinheiten übertragenen Temperaturistwerte speichert, vorzugsweise protokolliert. Insbesondere können die übertragenen Temperaturistwerte in einem zeitlichen voreingestellten Abstand, der jeweiligen aktuellen Uhrzeit zugeordnet gespeichert werden. Der voreingestellte zeitliche Abstand ist dabei auf einen Wert im Bereich zwischen 1 Sekunde und 5 Minuten, insbesondere auf einen Abstand von 10 Sekunden bis 60 Sekunden voreingestellt. Hierdurch wird der zeitliche Verlauf der Temperaturistwerte gespeichert. Dieser zeitliche Verlauf kann dabei insbesondere grafisch auf einer Anzeigeeinheit der zentralen Steuereinheit oder auf einer mit dieser verbundenen weiteren Datenverarbeitungseinheit, wie beispielsweise über die Anzeigeeinheit einer zentralen Behandlungsraum- oder Operationssaalsteuereinheit, erfolgen.

Weiterhin ist es vorteilhaft, wenn jede Postelementsteuereinheit eine Schutzschaltung und/oder eine Schutzfunktion umfasst, die eine Überhitzung des Heizelements und/oder des Polsterelements verhindert. Dadurch wird sichergestellt, dass keine Gefährdung des Patienten erfolgt und dass die Komponenten und Materialen des Postelements nicht durch zu große Hitzeeinwirkung geschädigt oder gar entzündet werden.

Weiterhin ist es vorteilhaft, dass jedes Polsterelement mindestens ein Schaumstoffformteil umfasst. Dadurch ist eine bequeme und einfache Lagerung des Patienten möglich. Die Form von Schaumstoffformteilen kann einfach den Erfordernissen angepasst werden. Ferner kann der Härtegrad von Schaumstoffformteilen einfach durch geeignete Auswahl des eingesetzten Schaumstoffs oder geeignete Fertigungsmethoden an die Erfordernisse des jeweiligen Polsterelements angepasst werden. Erfindungsgemäß hat zumindest das erste der mindestens zwei Polsterelemente einen ersten elektrischen Anschluss zum Zuführen zumindest der für die Heizelemente des ersten Polsterelements und des zweiten Polsterelements erforderlichen elektrischen Energie und mindestens einen zweiten elektrischen Anschluss zum Bereitstellen der für das Heizelement des zweiten Polsterelements erforderlichen elektrischen Energie. Dadurch kann die für das zweite Polsterelement erforderliche elektrische Energie einfach durch das erste Polsterelement hindurchgeleitet werden. Dadurch kann der Verdrahtungsaufwand zum Zuführen von elektrischer Energie für die Heizelemente und/oder die Polsterelementsteuereinheiten erheblich reduziert werden. Ferner kann die Handhabung der Polsterelemente erheblich vereinfacht werden.

Dabei ist es vorteilhaft, wenn der erste elektrische Anschluss zusätzlich zum Zuführen von Steuersignalen und/oder von Steuerdaten zumindest zum Übertragen des mindestens einen Temperatursollwerts zu der in das erste Polsterelement integrierten ersten Polsterelementsteuereinheit dient. Der zweite elektrische Anschluss dient dann vorzugsweise zusätzlich zum Bereitstellen von Steuersignalen und/oder Steuerdaten zumindest zum Übertragen des mindestens einen Temperatursollwerts zu der in das zweite Polsterelement integrierten zweiten Polsterelementsteuereinheit. Dadurch können über dieselben elektrischen Anschlüsse sowohl die für die elektrischen Heizelemente erforderliche elektrische Energie als auch die von der zentralen Steuereinheit zu den Polsterelementsteuereinheiten zu übertragenden Steuersignale und/oder Steuerdaten übertragen werden, sodass zum Herstellen der elektrischen Verbindungen vorzugsweise nur ein elektrischer Steckverbinder zwischen den Polsterelementen vorgesehen werden muss. Dabei kann die Übertragung der Steuersignale und/oder Steuerdaten auch über dieselben Drähte erfolgen, wie die Energieversorgung der Polsterelemente. Die Steuerdaten können beispielsweise auf die Versorgungsspannung aufmoduliert werden, beispielsweise in Form einer Powerline-Datenübertragung.

Ferner ist es vorteilhaft, wenn die erste elektrische Verbindung und/oder die zweite elektrische Verbindung durch einen elektrischen Steckverbinder mit mindestens zwei elektrischen Kontakten, vorzugsweise mit zwei elektrischen Kontakten für das Zuführen der elektrischen Energie zum Erzeugen der Heizleitung der elektrischen Heizelemente und zwei elektrischen Kontakte zum Zuführen der Steuersignale bzw. Steuerdaten, hergestellt werden. Hierdurch können einfach die erforderlichen elektrischen Verbindungen zu den Polsterelementen hergestellt werden. Alternativ zur Steuersignalübertragung oder Steuerdatenübertragung über die erste und/oder zweite elektrische Verbindung können diese auch drahtlos, beispielsweise über Infrarot, WLAN-Netzwerke oder Bluetooth-Datenverbindungen, übertragen werden.

Besonders vorteilhaft ist es, wenn die elektrische Verbindung der Polsterelemente seriell und/oder sternförmig erfolgt. Dadurch ist eine einfache Verbindung sowohl der Polsterelemente mit der zentralen Steuereinheit als auch der Polsterelemente untereinander einfach möglich. Der Verdrahtungsaufwand zum Verbinden der Polsterelemente mit der zentralen Steuereinheit und mit der zentralen Energieversorgung kann dadurch erheblich reduziert und die Handhabung der Polsterelemente vereinfacht werden. Die serielle Verbindung von mindestens zwei Polsterelementen zum Durchschleifen von Versorgungsleitungen zum Zuführen von elektrischer Energie und von Datenleitungen zum Zuführen von Steuerdaten oder zum Durchschleifen von Steuersignalen wird auch als Daisy Chain bezeichnet. Als Daisy Chain bezeichnet man eine Anzahl von Hardwarekomponenten, welche in Serie miteinander verbunden sind, vorzugsweise in sogenannten Bussystemen, wie sie aus der Automatisierungs- und Automobiltechnik bekannt sind. Dabei ist die erste Komponente direkt mit der zentralen Steuereinheit verbunden. Die weiteren Komponenten sind jeweils mit ihren Vorgängern verbunden. Die so entstehende Kette wird als Daisy Chain bezeichnet. Die Komponenten der erfindungsgemäßen Anordnung sind die Polsterelementsteuereinheiten und/oder die Heizelemente der Polsterelemente.

Ferner ist es vorteilhaft, wenn ein Bussystem zum Übertragen von Daten zwischen der zentralen Steuereinheit und den Polsterelementsteuereinheiten vorgesehen ist, über das zumindest der Temperatursollwert bzw. die Temperatursollwerte von der zentralen Steuereinheit zu dem Polsterelementsteuereinheiten und/oder die ermittelten Temperaturwerte von den Polsterelementsteuereinheiten zur zentralen Steuereinheit übertragen werden. Als Bussystem wird vorzugsweise ein CAN-Bussystem eingesetzt. Dabei können die mit Hilfe des Bussystems übertragenen Steuerdaten und/oder Steuersignale zusätzlich oder alternativ Fehlersignale, Programmdaten Freigabesignale und/oder weitere Betriebsparameter der zentralen Steuereinheit und/oder der Polsterelementsteuereinheiten umfassen. Die Steuerdaten und/oder Steuersignale können vorzugsweise mit Hilfe von auf die Versorgungsspannung aufmodulierten Signalen übertragen werden. Dadurch ist eine besonders einfache und kostengünstige Verbindung der Polsterelemente mit der zentralen Steuereinheit zum Übertragen der Steuerdaten bzw. der Steuersignale möglich, da lediglich die Verbindung der Polsterelemente mit der Versorgungsspannung hergestellt werden muss. Alternativ können die Steuerdaten und/oder Steuersignale über separate Leitungen übertragen werden.

Ein zweiter Aspekt der Erfindung betrifft einen Operationstisch mit einer Patientenlagerfläche, die mindestens eine erste verstellbare Komponente und eine zweite verstellbare Komponente umfasst. Der Operationstisch hat ferner eine Anordnung nach Anspruch 1 oder einer der zuvor genannten Weiterbildungen der Erfindung. Die erste Komponente der Patientenlagerfläche umfasst das erste Polsterelement und die zweite Komponente der Patientenlagerfläche umfasst das zweite Polsterelement. Die Polsterelemente der Patientenlagerfläche des Operationstischs können dadurch sicher gehandhabt und einfach durch den Einsatz der erfindungsgemäßen Polsterelemente installiert werden.

Besonders vorteilhaft ist es, wenn bei dem Operationstisch das erste Polsterelement lösbar mit einem Tragrahmen oder einer Tragplatte der ersten Komponente verbindbar ist, und wenn das zweite Polsterelement lösbar mit einem Tragrahmen oder einer Tragplatte der zweiten Komponente verbindbar ist. Dadurch ist eine einfache Handhabung und Verbindung der Polsterelemente mit dem Operationstisch möglich.

Ferner ist es vorteilhaft, wenn bei dem Operationstisch die Tragrahmen und/oder Tragplatten zum Bereitstellen der für die elektrischen Heizelemente erforderlichen Energie und zum Übertragen des mindestens einen Temperatursollwerts elektrisch miteinander verbunden sind. Die elektrische Verbindung ist vorzugsweise lösbar ausgeführt, insbesondere über eine geeignete Steckverbinderanordnung. Die Steckverbinderanordnung ist hierzu vorzugsweise in mechanische Verbinder zum Verbinden der Komponenten derart integriert, dass eine elektrische Verbindung über die Steckverbinderanordnung beim Herstellen der mechanischen Verbindung automatisch erfolgt.

Die Polsterelemente sind mit dem Tragrahmen über mindestens eine lösbare elektrische Steckverbindung oder alternativ über eine induktive Verbindung zum Bereitstellen der für die elektrischen Heizelemente erforderlichen elektrischen Energie und zum Übertragen zumindest des mindestens einen Temperatursollwerts verbunden. Dadurch ist eine einfache Installation und Handhabung der erfindungsgemäßen Polsterelemente möglich, sodass die Polsterelemente der Patientenlagerfläche einfach erwärmt werden können. Weiterhin ist es vorteilhaft, wenn der Operationstisch mindestens einen Akkumulator zumindest zur kurzzeitigen Energieversorgung mindestens eines der elektrischen Heizelemente und zumindest der dieses Polsterelement ansteuernden Polsterelementsteuereinheit ist, der in die Patientenlagerfläche, in das jeweilige Polsterelement, in die zentrale Steuereinheit, in einem Transportwagen zum Transport der Patientenlagerfläche und/oder in einer Operationstischsäule des Operationstischs integriert ist. Dadurch wird erreicht dass das Zuführen von Wärme zu einem auf der Patientenlagerfläche lagernden Patienten auch dann möglich ist, wenn der Operationstisch nicht mit elektrischer Energie aus einem Versorgungsnetz versorgt werden kann, beispielsweise, wenn die Patientenlagerfläche separat von der Operationstischsäule transportiert wird.

Wie bereits erwähnt, regeln die Polsterelementsteuereinheiten abhängig von einem durch den Temperatursensor ermittelten Temperaturistwert und dem vorgegebenen Temperatursollwert die Wärmeabgabe des Heizelements des Polsterelements. Unter Regelung wird ein Vorgang verstanden, bei dem der prinzipiell veränderliche Temperaturistwert automatisch konstant oder annähernd konstant auf dem Temperatursollwert gehalten wird. Der Temperaturistwert wird mit Hilfe des Temperatursensors als Messwert ermittelt. Bei einer Abweichung des Temperaturistwerts vom Temperatursollwert (Regelabweichung) wird die von dem Heizelement des jeweiligen Polsterelements abgegebene Heizenergie abhängig von der ermittelten Regelabweichung verändert, sodass sich der Temperaturistwert an den Temperatursollwert annähert. Allgemein wird unter Regeln bzw. Regelung ein Vorgang verstanden, bei dem fortlaufend eine variable Größe (Temperaturistwert) erfasst und mit einer anderen variablen Größe (Temperatursollwert) verglichen und im Sinne einer Angleichung an die andere variable Größe beeinflusst wird. Die erfasste variable Größe wird auch als Regelgröße bezeichnet und die andere variable Größe als Führungsgröße. Ein Kennzeichen für das Regeln bzw. für die Regelung ist der geschlossene Wirkungskreislauf, bei dem die Regelgröße im Regelkreis fortlaufend sich selbst beeinflusst.

Besonders vorteilhaft ist es, wenn die zentrale Steuereinheit den Temperatursollwert bzw. die Temperatursollwerte der Kontaktoberfläche des Polsterelements zum Kontakt mit einem Patienten abhängig von der Körpertemperatur des Patienten automatisch in einem voreingestellten Temperaturbereich von vorzugsweise 24°C kleiner 41°C einstellt. Ferner ist es dabei vorteilhaft, wenn der Temperatursensor vonzurgsweise die Temperatur an der Kontaktoberfläche erfasst.

Weitere Merkmale und Vorteile der Erfindung ergeben sich aus der nachfolgenden Beschreibung, die die Erfindung anhand von Ausführungsbeispielen im Zusammenhang mit den beigefügten Figuren näher erläutert.

Es zeigen:
- Figur 1: den oberen Abschnitt einer Operationstischsäule mit einer mit der Operationstischsäule verbundenen Patientenlagerfläche;
- Figur 2: ein Transportwagen mit der von der Operationstischsäule getrennten Patientenlagerfläche nach Figur 1;
- Figur 3: ein mit der Operationstischsäule verbundenes Mitteilsegment und insgesamt vier weiteren mit diesen Mittelsegment verbindbaren Segmenten der Patientenlagerfläche, wobei die Polsterelemente des Mittelsegment und einem weiteren Segment separat dargestellt worden sind;
- Figur 4: eine schematische Darstellung des Aufbaus der Polsterelemente und der Verbindung der Polsterelemente mit einer zentralen Energieversorgungseinheit und einer zentralen Steuereinheit gemäß einer ersten Ausführungsform; und
- Figur 5: eine schematische Darstellung des Aufbaus der Polsterelemente und der Verbindung der Polsterelemente mit einer zentralen Energieversorgungseinheit und einer zentralen Steuereinheit gemäß einer zweiten Ausführungsform.

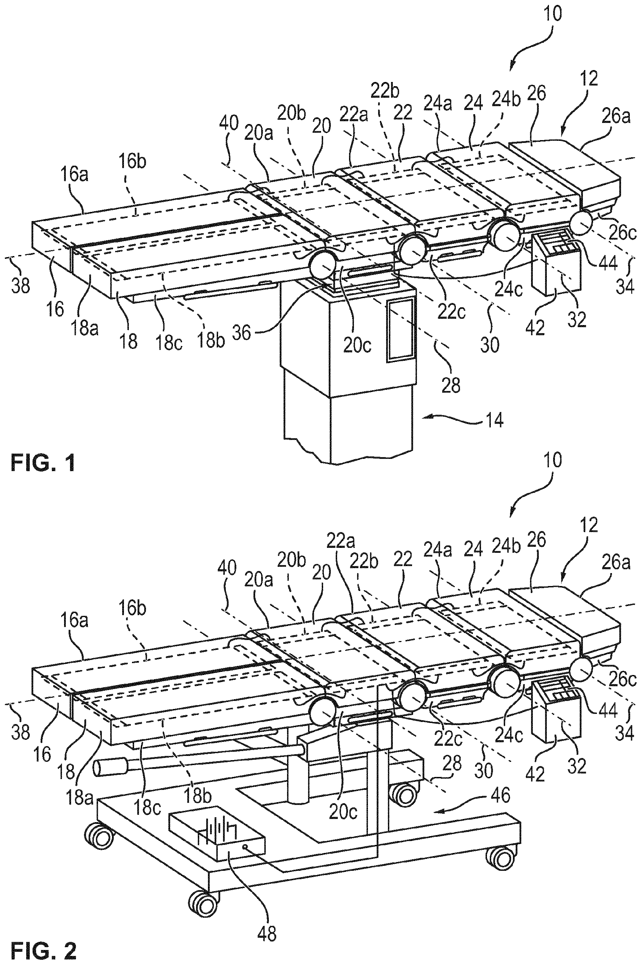

Figur 1 zeigt den oberen Teil eines Operationstischs 10, von dem der obere Abschnitt einer Operationstischsäule 14 und einer mit der Operationstischsäule 14 verbundene Patientenlagerfläche 12 dargestellt ist. Die Patientenlagerfläche 12 umfasst im vorliegenden Ausführungsbeispiel sechs Komponenten, nämlich zwei Beinplatten 16, 18, eine zentrale Mittelplatte 20, zwei Rumpfplatten 22, 24 und eine Kopfplatte 26. Die Komponenten 16 bis 26 sind im vorliegenden Ausführungsbeispiel über elektromotorische Antriebe um Drehachsen 28, 30, 32, 34 verschwenkbar zueinander angeordnet. Weiterhin ist die Patientenlagerfläche 12 über entsprechende Schwenkantriebe eines den oberen Abschnitt der Operationstischsäule 14 bildenden Operationstischsäulenkopfs 36 um ihre Längsachse 38 und ihre Querachse 40 schwenkbar angeordnet. Alle Komponenten 16 bis 26 umfassen eine Grundplatte und/oder einen Grundrahmen auf denen jeweils mindestens ein Polsterelement mit mindestens einem in das jeweilige Polsterelement integrierten Heizelement haben. Im vorliegenden Ausführungsbeispiel ist jeweils nur eine in jedes Polsterelement integrierte Heizmatte als Strichlinie dargestellt. Die Kopfplatte 26 hat im vorliegenden Ausführungsbeispiel ein Polster, in das kein Heizelement integriert ist. Im Folgenden werden die Polsterelemente der jeweiligen Komponente mit der Bezugszeichenziffer der jeweiligen Komponente 16 bis 26 den kleinen Buchstaben a, die in das Polsterelement integrierte Heizmatte mit der Bezugszeichenziffer der jeweiligen Komponente 16 bis 26 und dem kleinen Buchstaben b sowie der Tragrahmen bzw. Tragplatte mit der Bezugszeichenziffer der jeweiligen Komponente und dem kleinen Buchstaben c bezeichnet. Die Beinplatte 18 umfasst somit das Polsterelement 18a, die Heizmatte 18b und den Tragrahmen 18c.

Der Operationstisch 10 umfasst eine zentrale Steuereinheit 42, die über eine nicht dargestellte Steckverbinderanordnung vorzugsweise über die Trageplatte 20c oder direkt mit dem Polsterelement 20a verbunden ist. Ferner ist die zentrale Steuereinheit 42 über eine nicht dargestellte Verbindung mit einem Stromversorgungsnetzt eines Operationssaals verbunden. Die Polsterelemente 16a bis 24a sind mit Hilfe von Steckverbindern lösbaren Kabelbrücken elektrisch miteinander verbunden, sodass über diese Kabelbrücken sowohl die Versorgungsspannung als auch Steuersignale und/oder Steuerdaten bidirektional zwischen der zentralen Steuereinheit 42 und in jedes der Polsterelemente 16a bis 24a integrierte Polsterelementsteuereinheiten (nicht dargestellt) verbunden ist. Über eine Mensch-Maschinen-Schnittstelle, d. h. über ein Userinterface 44, der zentralen Steuereinheit 42 kann die Heizfunktion der in die Polsterelemente 16a bis 24a integrierten Heizmatten 16b bis 24b aktivieren und mindestens einen Temperatursollwert vorgeben, auf den die mit Hilfe eines in jedes der Polsterelemente 16a bis 24a integrierten Temperatursensors detektierten Polsteristtemperatur auf einen für dieses Polsterelement 16a bis 24a an der zentralen Steuereinheit 42 eingestellten Temperatursollwert geregelt wird. Vorzugsweise werden über die bidirektionalen Datenverbindungen Störsignale, Fehlersignale und die mit Hilfe des jeweiligen Temperatursensors detektierten Temperaturistwerte an die zentrale Steuereinheit 42 übertragen und von dieser zusammen mit der aktuellen Detektionszeit protokolliert.

In Figur 2 ist ein Transportwagen 46 mit der von der Operationstischsäule 14 getrennten Patientenlagerfläche 12 nach Figur 1 dargestellt. Die Energieversorgung der Heizmatten 18b bis 24b, der Polsterelementsteuereinheiten und der zentralen Steuereinheit 42 ist nicht wie bei einer Verbindung der Patientenlagerfläche mit der Operationstischsäule 14 über ein Energieversorgungsnetz sondern über ein in den Transportwagen 46 integrierten Akkumulator 48 verbunden. Alternativ zum Akkumulator 48 kann auch eine Batterie vorgesehen sein. Bei einer anderen Ausführungsform kann alternativ oder zusätzlich eine Energieversorgungseinheit, wie eine Batterie oder ein Akkumulator, in der zentralen Steuereinheit 42 vorgesehen sein, wobei die Batterie oder der Akkumulator vorzugsweise auswechselbar sind.

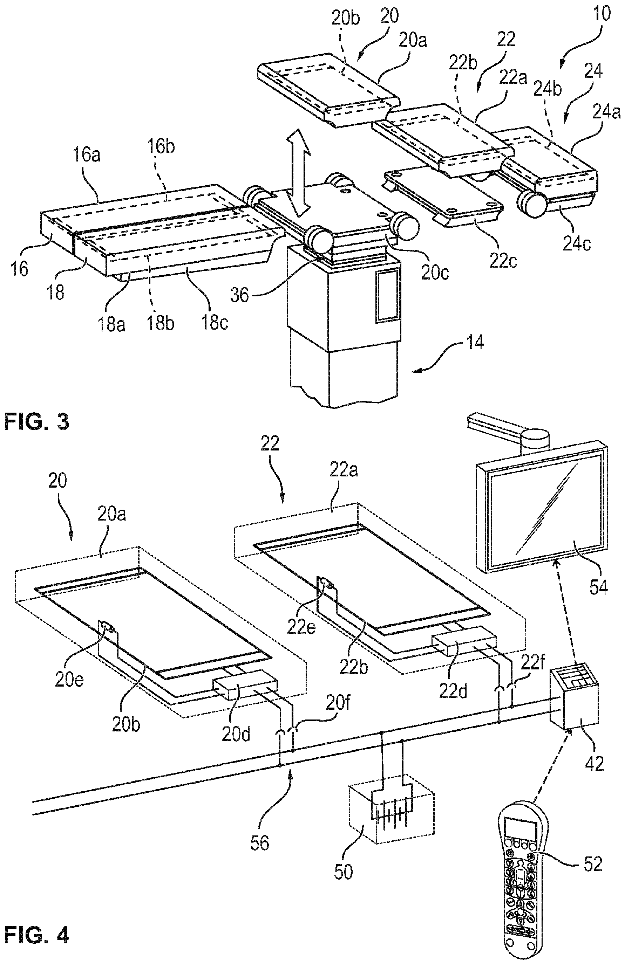

In Figur 3 ist die mit dem Operationstischsäulenkopf 36 der Operationstischsäule 14 verbundene Mittelplatte 20 mit dem von der Tragplatte 20c getrennten Polsterelement 20a gezeigt. Die weiteren Komponenten 16, 18, 22, 24, die Polsterelemente 16a, 18a, 22a, 24a mit dem Heizmatten 16b, 18b, 22b, 24b, sind getrennt voneinander und getrennt von der Mittelplatte 20 dargestellt. Die Polsterelemente 20a, 22a der Komponenten 20, 24 sind getrennt von den jeweiligen Tragplatten 20c, 22c gezeigt. Die Polsterelemente 20a bis 26a lassen sich über lösbare Verbindungen von den jeweiligen Tragplatten 20c bis 26c bzw. Tragrahmen trennen sodass sie einfach gereinigt und desinfiziert werden können. Ferner können die von den Tragplatten 20c bis 26c gelösten Polsterelemente einfach gegen andere Polsterelemente ausgetauscht und einfach transportiert und aufbewahrt, insbesondere übereinander gestapelt aufbewahrt, werden.

Figur 4 zeigt eine schematische Darstellung des Aufbaus der Polsterelemente 20a, 22a und der Verbindung der Polsterelemente 20a, 22a über ein Zweidraht-Bussystem mit einer zentralen Spannungsversorgungseinheit 50 und der zentralen Steuereinheit 42. Bei allen Ausführungsbeispielen können Bedieneingaben alternativ oder zusätzlich zu dem Userinterface 44 der zentralen Steuereinheit 42 auch über eine drahtlose oder drahtgebundene Fernbedienung 52 oder über eine drahtlose oder drahtgebundene mit der zentralen Steuereinheit 42 verbundene zentrale Steuereinheit 54 erfolgen, wobei mit Hilfe der zentralen Steuereinheit 54 vorzugsweise eine Vielzahl von Steuerfunktionen im Operationssaal aktiviert und überwacht werden können, beispielsweise die Lichtsteuerung, die Klimasteuerung, die Steuerung des Operationstischs und weiterer medizinischer Geräte und Apparate im Operationsraum.

Im Inneren des Polsterelements 20a sind neben der Heizmatte 20c, mindestens ein Temperatursensor 20e und eine Polsterelementsteuereinheit 20d angeordnet. Die Polsterelementsteuereinheit 20d ist über eine Steckverbinderanordnung 20f mit dem Zweidraht-Bussystem 56, über das sowohl Steuerdaten zwischen dem Polsterelementsteuereinheiten 20d, 22d und der zentralen Steuereinheit 42 übertragen werden als auch die Versorgungsspannung für die Polsterelementsteuereinheiten 20d, 22d, die Temperatursensoren 20e, 22e und die Heizelemente 20c, 22c bereitgestellt wird, verbunden. Die Polsterelemente 16a, 18a, 24a der weiteren Komponenten haben in gleicher Weise wie die Polster 20a, 22a jeweils eine Polsterelementsteuereinheit 16d, 18d, 24d, mindestens einen Temperatursensor 16e, 18e, 24e und mindestens eine Steckverbinderanordnung 16f, 18f, 24f.

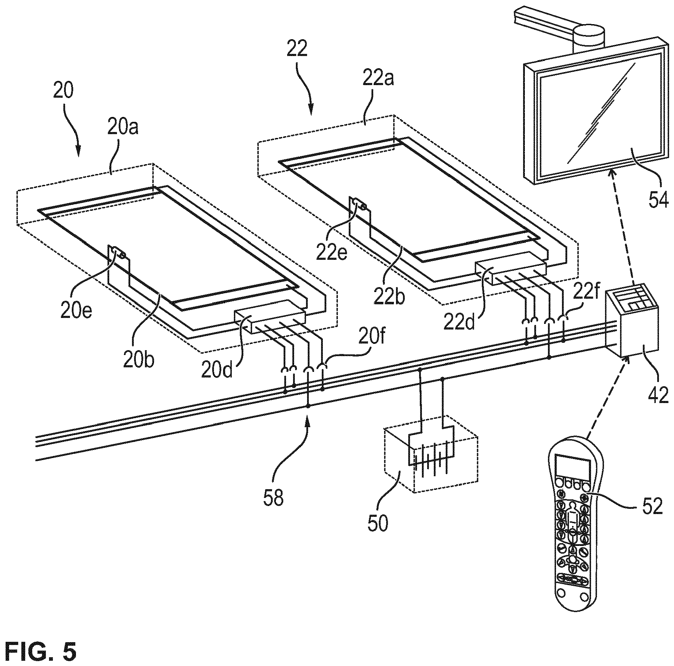

Figur 5 zeigt eine schematische Darstellung eines alternativen Aufbaus der Polsterelemente 20a, 22a und einer zu der ersten Ausführung nach Figur 4 alternativen Verbindungen der Polsterelemente 20a, 22a mit der zentralen Energieversorgungseinheit 50 und der zentralen Steuereinheit 42 gemäß einer zweiten Ausführungsform. Bei dieser zweiten Ausführungsform wird anstatt eines Zweidraht-Bussystems 56 ein Vierdraht-Bussystem, bei dem die Steuerdaten bzw. die Steuersignale zwischen der zentralen Steuereinheit 42 und den Polsterelementsteuereinheiten 20d, 22d über separate Leitungen erfolgt, sodass diese Signalleitungen entkoppelt von den Energieversorgungsleitungen sind. Dadurch ist eine höhere Übertragungsgeschwindigkeit und eine geringere Störung der übertragenen Steuersignale und/oder Steuerdaten möglich. Zur Vereinfachung sind die Polsterelemente, Temperatursensoren, Heizmatten, Polsterelementsteuereinheiten und die zentrale Steuereinheit bei der zweiten Ausführungsform mit den gleichen Bezugszeichen bezeichnet wie bei der ersten Ausführungsform.

Sowohl bei der ersten Ausführungsform nach Figur 4 als auch bei der zweiten Ausführungsform nach Figur 5 sind die weiteren mit Heizmatten versehenen Polsterelemente 16, 18, 24 in gleicher Weise aufgebaut, wie dies in den Figuren 4 und 5 für die Polsterelemente 20a, 22a gezeigt worden ist. Auch sind die Polsterelementsteuereinheiten 16d, 18d, 24d dieser Polsterelemente 16a, 18a, 24a mit den jeweiligen Bussystemen 56, 58 verbunden.

Bei allen Ausführungsformen können weitere Temperatursensoren vorgesehen sein, um die Temperatur an verschiedenen Stellen des jeweiligen Polsterelements 16a bis 24a zu detektieren. Vorzugsweise ist mindestens ein oberflächennaher Sensor 16e bis 24e und jeweils ein in unmittelbarer Nähe zur Heizmatte angeordneter zweiter Temperatursensor vorgesehen, mit dessen Hilfe eine Überhitzung der jeweiligen Heizmatte detektierbar ist.

Als Temperatursensoren werden insbesondere Zweidraht-Temperatursensoren eingesetzt, wie z. B. ein PT100 Widerstandselement PTC-Thermistoren oder NTC-Thermistoren, insbesondere PTC100-Thermistoren oder NTC100-Thermistoren. Die Heizleistung der Heizmatten ist vorzugsweise derart dimensioniert, dass die Oberflächenheizleistung der Polsterelemente 16a bis 24a <= 115 W/m² ist.

Die Polsterelementsteuereinheiten 20d, 22d regeln den Temperaturistwert auf den voreingestellte Temperatursollwert nach Art eines geschlossenen Regelkreises.

Je nach Ausführungsform kann die zentrale Steuereinheit 42 den Polsterelementsteuereinheiten 16d bis 24d jeweils denselben Temperatursollwert oder jeder Polsterelementsteuereinheit 16d bis 24d einen individuellen Temperatursollwert vorgeben. Jedes Polsterelement 16a bis 26a hat vorzugsweise mindestens ein Schaumstoffformteil.

Mit Hilfe des in unmittelbarer Nähe der Heizmatte 16b bis 24b angeordneten Temperatursensors wird die Temperatur der Heizmatte 16b bis 24b detektiert. Der detektierte Temperaturwert wird von einer Schutzschaltung und/oder einer Schutzfunktion der Polsterelementsteuereinheit verarbeitet, wobei eine Überhitzung der Heizmatte 16b bis 24b und/oder des Polsterelements 16a bis 24a detektiert und beispielsweise durch eine Abschaltung der Heizmatten 16b bis 24b verhindert wird.

Als Bussystem wird vorzugsweise ein CAN-Bussystem eingesetzt, durch das eine einfache und sichere Datenübertragung zwischen der zentralen Steuereinheit 42 und den Polsterelementsteuereinheiten 16d bis 24d sichergestellt ist. Die Übertragung von Daten kann alternativ zu den angegebenen drahtgebundenen Lösungen auch über eine drahtlose Datenübertragungsverbindung, beispielsweise über WIFI, Bluetooth oder Infrarot erfolgen. Ferner kann die Datenübertragung und/oder die Energieübertragung auch induktiv zwischen dem jeweiligen Polsterelement 16a bis 24a und der Tragplatte 16c bis 24c bzw. Tragrahmen auf dem das Polsterelement 16a bis 24a aufliegt, erfolgen. Hierzu sind dann die Tragrahmen und/oder Tragplatten 16c bis 24c der Komponenten 16 bis 24 der Patientenlagerfläche 12 über entsprechende Verbindungen elektrisch miteinander verbunden.

Aus Sicherheitsgründen kann die Spannungsversorgung der Heizmatten 16b bis 24b mit einer Betriebsspannung kleiner der Netzspannung erfolgen, beispielsweise mit Schutzkleinspannung. Die zentrale Steuereinheit 42 und/oder die jeweilige Polsterelementsteuereinheit 16d bis 24d kann zusätzlich einen Akkumulator umfassen, der bei Trennung der zentralen Steuereinheit 42 bzw. der Polsterelementsteuereinheiten 16d bis 24d von einem Versorgungsnetz die Energieversorgung der Heizmatte 16b bis 24 b übernimmt. Vorzugsweise ist dieser Akkumulator in die jeweilige Steuereinheit 42, 16d bis 24d integriert und wird über eine entsprechende Ladeschaltung geladen, solange die Steuereinheit 42, 16d bis 24d mit einem Versorgungsnetz verbunden ist.

Anstatt der in den Ausführungsbeispielen erwähnten Heizmatten 16b bis 24b können auch andere Heizelemente, insbesondere Widerstandsheizelemente, vorzugsweise in Decken integrierte Widerstandsheizelemente, eingesetzt werden.

Besonders vorteilhaft ist es, wenn die zentrale Steuereinheit (42) den Temperatursollwert bzw. die Temperatursollwerte abhängig von der Körpertemperatur des Patienten automatisch in einem voreingestellten Temperaturbereich von vorzugsweise 24°C bis 37°C einstellt.

### Bezugszeichenliste

- 10: Operationstisch
- 12: Patientenlagerfläche
- 14: Operationstischsäule
- 16, 18: Beinplatte
- 16a bis 26a: Polsterelement
- 16b bis 24b: Heizmatte
- 16c bis 26c: Tragplatte
- 16d bis 24d: Polsterelementsteuereinheit
- 16e bis 24e: Temperatursensor
- 16f bis 24f: Steckverbinder
- 20: Mittelsegment
- 22, 24: Rückensegment
- 26: Kopfplatte
- 28, 30, 32, 34: Drehachsen
- 36: Operationstischsäulenkopf
- 38: Längsachse der Patientenlagerfläche
- 40: Querachse der Patientenlagerfläche
- 42: zentrale Steuereinheit
- 44: Mensch-Maschine-Schnittstelle
- 46: Transportwagen
- 48: Akkumulator
- 50: Energieversorgungseinheit
- 52: Fernbedienung
- 54: zentrale Bedieneinheit Operationssaal
- 56: Zweidraht-Bussystem
- 58: Vierdraht-Bussystem

## Patentansprüche

1. Anordnung zum Erwärmen einer Patientenlagerfläche, insbesondere zum Erwärmen von Polsterelementen (16a bis 24a) für eine Patientenlagerfläche (12) eines Operationstischs (10),
mit einem ersten Polsterelement (20a) und mit mindestens einem zweiten Polsterelement (22a), die jeweils ein im Polsterelement (20a, 22a) integriertes elektrisches Heizelement (20b, 22b) haben,
wobei eine zentrale Energieversorgungseinheit (48, 50) zum Versorgen der elektrischen Heizelemente (20b, 22b) mit elektrischer Energie vorgesehen ist und eine zentrale Steuereinheit (42) zum Steuern der Wärmeabgabe der Patientenlagerfläche (12) vorgesehen ist,
**dadurch gekennzeichnet, dass** in jedem Polsterelement (20a, 22a) eine Polsterelementsteuereinheit (20d, 22d) zum Steuern der Wärmeabgabe des Heizelements (20b, 22b) des Polsterelements (20a, 22a) und mindestens ein Temperatursensor (20e, 22e) integriert sind,
dass die zentrale Steuereinheit (42) den Polterelementsteuereinheiten (20d, 22d) mindestens einen Temperatursollwert vorgibt, und
dass jede Polsterelementsteuereinheit (20d, 22d) ausgehend von einem durch den jeweiligen Temperatursensor (20e, 22e) ermittelten Temperaturistwert und dem vorgegebenen Temperatursollwert die Wärmeabgabe des Heizelements (20b, 22b) des Polsterelements (20a, 22a) regelt, und
dass zumindest das erste der mindestens zwei Polsterelemente (20a, 22a) einen ersten elektrischen Anschluss zum Zuführen zumindest der für die Heizelemente (20b, 22b) des ersten Polsterelements (20a) und des zweiten Polsterelements (22a) erforderlichen elektrischen Energie und mindestens einen zweiten elektrischen Anschluss zum Bereitstellen der für das Heizelement (22b) des zweiten Polsterelements (22a) erforderlichen elektrischen Energie hat.

2. Anordnung nach Anspruch 1, **dadurch gekennzeichnet, dass** die zentrale Steuereinheit beiden Polsterelementsteuereinheiten (20d, 22d) denselben Temperatursollwert oder jeder Polsterelementsteuereinheit (20d, 22d) einen individuellen Temperatursollwert vorgibt.

3. Anordnung nach einem der vorhergehenden Ansprüche, **dadurch gekennzeichnet, dass** jede Polsterelementsteuereinheit (20d, 22d) den mit Hilfe des Temperatursensors (20e, 22e) ermittelten Temperaturistwert zur zentralen Steuereinheit (42) überträgt.

4. Anordnung nach Anspruch 3, **dadurch gekennzeichnet, dass** die zentrale Steuereinheit (42) zumindest einen Teil der von den Polsterelementsteuereinheiten (20d, 22d) übertragenen Temperaturistwerten speichert.

5. Anordnung nach einem der vorhergehenden Ansprüche, **dadurch gekennzeichnet, dass** die Polsterelementsteuereinheit (20d, 22d) eine Schutzschaltung und/oder eine Schutzfunktion umfasst, die eine Überhitzung des Heizelements (20b, 22b) und/oder des Polsterelements (20a, 22a) verhindert.

6. Anordnung nach einem der vorhergehenden Ansprüche, **dadurch gekennzeichnet, dass** jedes Polsterelement (20a, 22a) mindestens ein Schaumstoffformteil umfasst.

7. Anordnung nach einem der vorhergehenden Ansprüche, **dadurch gekennzeichnet, dass** der erste elektrische Anschluss zusätzlich zum Zuführen von Steuersignalen und/oder Steuerdaten zumindest zum Übertragen des mindestens einen Temperatursollwerts zu der in das erste Polsterelement (20a) integrierten ersten Polterelementsteuereinheit (20d) dient und dass der zweite elektrische Anschluss zum Bereitstellen von Steuersignalen und/oder Steuerdaten zumindest zum Übertragen des mindestens einen Temperatursollwerts zu der in das zweite Polsterelement (22a) integrierten zweiten Polsterelementsteuereinheit (22d) dient.

8. Anordnung nach einem der vorhergehenden Ansprüche, **dadurch gekennzeichnet, dass** die erste elektrische Verbindung und/oder die zweite elektrische Verbindung durch einen elektrischen Steckverbinder mit mindestens zwei elektrischen Kontakten, vorzugsweise mit zwei elektrischen Kontakten für das Zuführen der elektrischen Energie für die elektrischen Heizelemente (20b, 22b) und zwei elektrischen Kontakten zum Zuführen der Steuersignale bzw. Steuerdaten, hergestellt wird.

9. Anordnung nach einem der vorhergehenden Ansprüche, **dadurch gekennzeichnet, dass** die elektrische Verbindung der Polsterelemente (16a bis 24a) seriell und/oder sternförmig erfolgt.

10. Anordnung nach einem der vorhergehenden Ansprüche, **dadurch gekennzeichnet, dass** ein Bussystem (56, 58) zum Übertragen von Daten zwischen der zentralen Steuereinheit (42) und den Polsterelementsteuereinheiten (20d, 22d) vorgesehen ist, über das zumindest der Temperatursollwert bzw. die Temperatursollwerte von der zentralen Steuereinheit (42) zu den Polsterelementsteuereinheiten (20d, 22d) und/oder die ermittelten Temperaturwerte von den Polsterelementsteuereinheiten (20d, 22d) zur zentralen Steuereinheit (42) übertragen werden.

11. Anordnung nach einem der vorhergehenden Ansprüche, **dadurch gekennzeichnet, dass** mindestens ein Akkumulator (48, 50) zumindest zur kurzzeitigen Energieversorgung mindestens eines der elektrischen Heizelemente (20b, 22b) und zumindest der dieses Heizelement (20b, 22b) ansteuernden Polsterelementsteuereinheit (20d, 22d) vorgesehen ist, der in die Patientenlagerfläche (12), in das jeweilige Polsterelement (20a, 22a), in einen Transportwagen (46) zum Transport der Patientenlagerfläche (12) und/oder in eine Operationstischsäule (14) des Operationstischs (10) integriert ist.

12. Operationstisch mit einer Patientenlagerfläche (12), die mindestens eine erste und eine zweite verstellbare Komponente (16 bis 26) umfasst, mit einer Anordnung nach einem der vorhergehenden Ansprüche, wobei eine erste Komponente (20) das erste Polsterelement (20a) und eine zweite Komponente (22) das zweite Polsterelement (22a) umfasst.

13. Operationstisch nach Anspruch 12, **dadurch gekennzeichnet, dass** das erste Polsterelement (20a) lösbar mit einem Tragrahmen oder einer Trageplatte (20c) der ersten Komponente (20) mechanisch verbindbar ist, und dass das zweite Polsterelement (22a) lösbar mit einem Tragrahmen oder einer Tragplatte (22c) der zweiten Komponente (22) mechanisch verbindbar ist.

14. Operationstisch nach Anspruch 13, **dadurch gekennzeichnet, dass** die Tragrahmen und/oder die Tragplatten (16c bis 24c) zum Bereitstellen der für die elektrischen Heizelemente (16b bis 24b) erforderlichen Energie und zum Übertragen des mindestens einen Temperatursollwerts elektrisch verbunden sind, und
dass die Polsterelemente (16a bis 24a) mit dem jeweiligen Tragrahmen und/oder der jeweiligen Tragplatte (16c bis 24c) über mindestens eine lösbare elektrische Steckverbindung oder über eine induktive Verbindung zum Bereitstellen der für die elektrischen Heizelemente (16b bis 24b) erforderlichen Energie und zum Übertragen des mindestens einen Temperatursollwerts verbunden sind.

## Claims

1. A system for heating a patient support surface, in particular for heating cushion elements (16a through 24a) for a patient support surface (12) of an operating table (10),
having a first cushion element (20a) and at least one second cushion element (22a), each having an electrical heating element (20b, 22b) that is integrated into the cushion element (20a, 22a),
wherein a central energy supply unit (48, 50) is provided for supplying the electrical heating elements (20b, 22b) with electrical energy, and a central control unit (42) is provided for controlling the heat delivery to the patient support surface (12),
**characterized in that** a cushion element control unit (20d, 22d) for controlling the heat delivery of the heating element (20b, 22b) to the cushion element (20a, 22a), and at least one temperature sensor (20e, 22e) are integrated into each cushion element (20a, 22a),
the central control unit (42) specifies at least one target temperature value for the cushion element control units (20d, 22d), and
each cushion element control unit (20d, 22d), starting from an actual temperature value that is determined by the particular temperature sensor (20e, 22e) and the predefined target temperature value, regulates the heat delivery of the heating element (20b, 22b) to the cushion element (20a, 22a), and
at least the first of the at least two cushion elements (20a, 22a) has a first electrical connection for supplying at least the electrical energy required for the heating elements (20b, 22b) of the first cushion element (20a) and of the second cushion element (22a), and has at least one second electrical connection for providing the electrical energy required for the heating element (22b) of the second cushion element (22a).

2. The system according to Claim 1, **characterized in that** the central control unit specifies the same target temperature value for both cushion element control units (20d, 22d), or specifies an individual target temperature value for each cushion element control unit (20d, 22d).

3. The system according to one of the preceding claims, **characterized in that** each cushion element control unit (20d, 22d) transmits the actual temperature value, determined by means of the temperature sensor (20e, 22e), to the central control unit (42).

4. The system according to Claim 3, **characterized in that** the central control unit (42) stores at least a portion of the actual temperature values transmitted from the cushion element control units (20d, 22d).

5. The system according to one of the preceding claims, **characterized in that** the cushion element control unit (20d, 22d) includes a protective circuit and/or a protective function that prevent(s) overheating of the heating element (20b, 22b) and/or of the cushion element (20a, 22a).

6. The system according to one of the preceding claims, **characterized in that** each cushion element (20a, 22a) includes at least one foam molded part.

7. The system according to one of the preceding claims, **characterized in that** the first electrical connection is additionally used for supplying control signals and/or control data at least for transmitting the at least one target temperature value to the first cushion element control unit (20d) that is integrated into the first cushion element (20a), and the second electrical connection is used for providing control signals and/or control data at least for transmitting the at least one target temperature value to the second cushion element control unit (22d) that is integrated into the second cushion element (22a).

8. The system according to one of the preceding claims, **characterized in that** the first electrical connection and/or the second electrical connection are/is established by an electrical plug-in connector having at least two electrical contacts, preferably having two electrical contacts for supplying the electrical energy for the electrical heating elements (20b, 22b), and having two electrical contacts for supplying the control signals or control data.

9. The system according to one of the preceding claims, **characterized in that** the electrical connection of the cushion elements (16a through 24a) takes place in series and/or in a star shape.

10. The system according to one of the preceding claims, **characterized in that** a bus system (56, 58) is provided for transmitting data between the central control unit (42) and the cushion element control units (20d, 22d), via which at least the target temperature value or the target temperature values is/are transmitted from the central control unit (42) to the cushion element control units (20d, 22d), and/or via which the determined temperature values are transmitted from the cushion element control units (20d, 22d) to the central control unit (42).

11. The system according to one of the preceding claims, **characterized in that** at least one accumulator (48, 50) is provided at least for temporarily supplying energy to least one of the electrical heating elements (20b, 22b) and to at least the cushion element control unit (20d, 22d) that controls this heating element (20b, 22b), and is integrated into the patient support surface (12), into the particular cushion element (20a, 22a), into a transport car (46) for transporting the patient support surface (12), and/or into an operating table column (14) of the operating table (10).

12. An operating table having a patient support surface (12) that includes at least one first and one second adjustable component (16 through 26), with a system according to one of the preceding claims, wherein a first component (20) includes the first cushion element (20a), and a second component (22) includes the second cushion element (22a).

13. The operating table according to Claim 12, **characterized in that** the first cushion element (20a) is detachably mechanically connectable to a support frame or a support plate (20c) of the first component (20), and the second cushion element (22a) is detachably mechanically connectable to a support frame or a support plate (22c) of the second component (22).

14. The operating table according to Claim 13, **characterized in that** the support frames and/or the support plates (16c through 24c) for providing the energy required for the electrical heating elements (16b through 24b) and for transmitting the at least one target temperature values are electrically connected, and
the cushion elements (16a through 24a) are connected to the particular support frame and/or the particular support plate (16c through 24c) via at least one detachable electrical plug-in connection or via an inductive connection for providing the energy required for the electrical heating elements (16b through 24b) and for transmitting the at least one target temperature value.

## Revendications

1. Dispositif pour chauffer une surface de support pour patients, en particulier pour chauffer des éléments de rembourrage (16a à 24a) pour une surface de support pour patients (12) d'une table d'opération (10),
avec un premier élément de rembourrage (20a) et avec au moins un deuxième élément de rembourrage (22a) comportant respectivement un élément chauffant électrique (20b, 22b) intégré dans l'élément de rembourrage (20a, 22a),
dans lequel une unité d'alimentation en énergie centrale (48, 50) pour alimenter les éléments chauffants électriques (20b, 22b) en énergie électrique est prévue, et une unité de commande centrale (42) pour commander l'émission de chaleur de la surface de support pour patients (12) est prévue,
**caractérisé en ce que** dans chaque élément de rembourrage (20a, 22a), une unité de commande d'élément de rembourrage (20d, 22d) pour commander l'émission de chaleur de l'élément chauffant (20b, 22b) de l'élément de rembourrage (20a, 22a) et au moins un capteur de température (20e, 22e) sont intégrés,
**en ce que** l'unité de commande centrale (42) spécifie au moins une valeur de température de consigne pour les unités de commande d'élément de rembourrage (20d, 22d), et
**en ce que** chaque unité de commande d'élément de rembourrage (20d, 22d) régule l'émission de chaleur de l'élément chauffant (20b, 22b) de l'élément de rembourrage (20a, 22a) en partant d'une valeur de température réelle, déterminée par le capteur de température (20e, 22e) respectif, et de la valeur de température de consigne spécifiée, et
**en ce qu'**au moins le premier desdits au moins deux éléments de rembourrage (20a, 22a) présente une première connexion électrique pour amener au moins l'énergie électrique nécessaire aux éléments chauffants (20b, 22b) du premier élément de rembourrage (20a) et du deuxième élément de rembourrage (22a) et au moins une deuxième connexion électrique pour fournir l'énergie électrique nécessaire à l'élément chauffant (22d) du deuxième élément de rembourrage (22a) .

2. Dispositif selon la revendication 1, **caractérisé en ce que** l'unité de commande centrale spécifie la même valeur de température de consigne pour les deux unités de commande d'élément de rembourrage (20d, 22d) ou spécifie une valeur de température de consigne individuelle pour chaque unité de commande d'élément de rembourrage (20d, 22d).

3. Dispositif selon l'une quelconque des revendications précédentes, **caractérisé en ce que** chaque unité de commande d'élément de rembourrage (20d, 22d) transmet à l'unité de commande centrale (42) la valeur de température réelle déterminée à l'aide du capteur de température (20e, 22e).

4. Dispositif selon la revendication 3, **caractérisé en ce que** l'unité de commande centrale (42) mémorise au moins une partie des valeurs de température réelles transmises par les unités de commande d'élément de rembourrage (20d, 22d).

5. Dispositif selon l'une quelconque des revendications précédentes, **caractérisé en ce que** l'unité de commande d'élément de rembourrage (20d, 22d) comprend un circuit de protection et/ou une fonction de protection empêchant une surchauffe de l'élément chauffant (20b, 22b) et/ou de l'élément de rembourrage (20a, 22a).

6. Dispositif selon l'une quelconque des revendications précédentes, **caractérisé en ce que** chaque élément de rembourrage (20a, 22a) comprend au moins une pièce moulée en mousse.

7. Dispositif selon l'une quelconque des revendications précédentes, **caractérisé en ce que** la première connexion électrique sert de plus à amener des signaux de commande et/ou des données de commande au moins pour transmettre ladite au moins une valeur de température de consigne à la première unité de commande d'élément de rembourrage (20d) intégrée dans le premier élément de rembourrage (20a), et **en ce que** la deuxième connexion électrique sert à fournir des signaux de commande et/ou des données de commande au moins pour transmettre ladite au moins une valeur de température de consigne à la deuxième unité de commande d'élément de rembourrage (22d) intégrée dans le deuxième élément de rembourrage (22a).

8. Dispositif selon l'une quelconque des revendications précédentes, **caractérisé en ce que** la première connexion électrique et/ou la deuxième connexion électrique est/sont établie(s) par un connecteur électrique avec au moins deux contacts électriques, de préférence avec deux contacts électriques pour amener l'énergie électrique pour les éléments chauffants électriques (20b, 22b) et deux contacts électriques pour amener les signaux de commande ou les données de commande.

9. Dispositif selon l'une quelconque des revendications précédentes, **caractérisé en ce que** la connexion électrique des éléments de rembourrage (16a à 24a) s'effectue en série et/ou en étoile.

10. Dispositif selon l'une quelconque des revendications précédentes, **caractérisé en ce qu'**il est prévu un système de bus (56, 58) pour transmettre des données entre l'unité de commande centrale (42) et les unités de commande d'élément de rembourrage (20d, 22d) par lequel est/sont transmise(s) au moins la/les valeur(s) de température de consigne de l'unité de commande centrale (42) aux unités de commande d'élément de rembourrage (20d, 22d) et/ou les valeurs de température déterminées des unités de commande d'élément de rembourrage (20d, 22d) à l'unité de commande centrale (42).

11. Dispositif selon l'une quelconque des revendications précédentes, **caractérisé en ce qu'**au moins un accumulateur (48, 50) est prévu au moins pour une alimentation en énergie temporaire d'au moins l'un des éléments chauffants électriques (20b, 22b) et au moins de l'unité de commande d'élément de rembourrage (20d, 22d) excitant ledit élément chauffant (20b, 22b), ledit accumulateur étant intégré dans la surface de support pour patients (12), dans ledit élément de rembourrage (20a, 22a) respectif, dans un chariot de transport (46) pour transporter la surface de support pour patients (12) et/ou dans une colonne de table d'opération (14) de la table d'opération (10).

12. Table d'opération avec une surface de support pour patients (12) comprenant au moins un premier et un deuxième composant réglable (16 à 26), avec un dispositif selon l'une quelconque des revendications précédentes, dans laquelle un premier composant (20) comprend le premier élément de rembourrage (20a) et un deuxième composant (22) comprend le deuxième élément de rembourrage (22a).

13. Table d'opération selon la revendication 12, **caractérisée en ce que** le premier élément de rembourrage (20a) peut être relié mécaniquement de manière amovible à un cadre ou plateau de support (20c) du premier composant (20), et **en ce que** le deuxième élément de rembourrage (22a) peut être relié mécaniquement de manière amovible à un cadre ou plateau de support (22c) du deuxième composant (22).

14. Table d'opération selon la revendication 13, **caractérisée en ce que** les cadres et/ou plateaux de support (16c à 24c) sont reliés électriquement pour fournir l'énergie nécessaire aux éléments chauffants électriques (16b à 24b) et pour transmettre ladite au moins une valeur de température de consigne, et
**en ce que** les éléments de rembourrage (16a à 24a) sont reliés au cadre et/ou plateau de support respectif (16c à 24c) par au moins une fiche de connexion électrique amovible ou par une connexion inductive pour fournir l'énergie nécessaire aux éléments chauffants électriques (16b à 24b) et pour transmettre ladite au moins une valeur de température de consigne.
